# EUROPEAN PATENT APPLICATION

(11) **EP 4 585 148 A1**
(43) Date of publication of application: **16.07.2025**
(21) Application number: 24305058.0
(22) Date of filing: 09.01.2024
(51) Int. Cl.: A61B 5/16

(54) **SYSTEM AND METHOD FOR MONITORING COMPULSIVE BEHAVIORS**

(71) Applicant: INSTITUT DU CERVEAU ET DE LA MOELLE ÉPINIÈRE - ICM, 75013 Paris (FR); APHP (Assistance Publique - Hôpitaux de Paris), 75012 Paris (FR); Centre National de la Recherche Scientifique, 75016 Paris (FR); Institut National de la Santé et de la Recherche Médicale (INSERM), 75013 Paris (FR); SORBONNE UNIVERSITE, 75006 Paris (FR)
(72) Inventor: HARTMANN, Andreas, 75001 Paris (FR); CZERNECKI, Virginie, 92190 MEUDON (FR); NEGOVANSKA, Velina, 94210 Saint Maur des Fossés (FR)
(74) Representative: Icosa

(57) **Abstract**

The present invention relates to a system (1000) and method for monitoring at least one uncontrolled behavior, said at least one uncontrolled behavior being preceded by at least one premonitory urge experienced by a user (20), said system (1000) comprising a sensing device (11) including at least one motion sensor, said motion sensor being configured to deliver a motion signal (101) representative of at least one voluntary action performed by said user (20) in response to said at least one premonitory urge; said system (1000) further comprising at least one processor (30) configured to receive said motion signal (101) from said sensing device (11) and to determine, based on said motion signal (101), said at least one premonitory urge.

## Description

### FIELD OF INVENTION

The present invention pertains to the field of compulsive behavior therapy. In particular, the invention relates to a system and method for monitoring compulsive behaviors.

### BACKGROUND OF INVENTION

Patients suffering from uncontrolled behaviors such as compulsive behaviors (e.g. for instance tics, eating disorders, Trichotillomania, Obsessive Compulsive Disorder, Attention Deficit Hyperactivity Disorder, Body Dysmorphic Disorder, Tourette Syndrome or addictions including alcoholism, compulsive shopping, smoking, drug-abuse or gambling) or anxious behaviors (e.g. behaviors associated with an excessive and persistent stress that interferes with daily functioning such as Generalized Anxiety Disorder, Panic Disorder, Post-Traumatic Stress Disorder, phobia including acrophobia, agoraphobia, glossophobia, sociophobia, mysophobia or claustrophobia) face daily challenges related to their symptoms. These compulsive behaviors are generally associated with a premonitory urge.

A premonitory urge refers to a subjective sensation or feeling that precedes and/or triggers an uncontrolled behavior.

In the context of anxious behaviors, the primary characteristics of the premonitory urge is a feeling that precedes the onset of anxious thoughts or behaviors. For example, a person with social anxiety might experience anticipatory anxiety characterized by physical or psychological symptoms before a social event, feeling a heightened sense of nervousness or unease.

In the context of compulsive behaviors, the premonitory urge is often described by patients experiencing it as a specific uncomfortable or distressing sensation that builds up. Performing the compulsive behavior provides a temporary sense of relief, satisfaction, and reduces anxiety. This relief is not, however, a permanent solution, and the cycle of compulsive behaviors and relief can persist. The rituals associated with compulsive behaviors may become time-consuming, interfere with daily activities, and contribute to increased distress over time.

In this context, there is a need for systems that may assist a user suffering from uncontrolled behaviors in controlling the premonitory urges, while also helping him documenting the premonitory urges and uncontrolled behaviors.

Indeed, learning to control uncontrolled behaviors may involve implementing therapeutic interventions, such as cognitive-behavioral therapy (CBT).

CBT may include exposure and response prevention therapy wherein a patient is gradually confronted with uncontrolled behaviors-provoking situations and learns to tolerate the symptoms such as discomfort associated with the premonitory urge and, over time, experiences a reduction of the symptoms until a tolerable level is reached. This process is known as habituation.

Exercises and consultations with healthcare professionals are essential for progress. However, patients often encounter difficulties in maintaining a rigorous practice of their exercises outside the sessions with health care professionals. They may forget to perform them, lack motivation, not practice adequately because patients are disturbed with writing down timing instead of concentrating on controlling the premonitory urge, or even lose the information they need to communicate to their healthcare professional at the end of each exercise to evaluate progress in the therapy.

Therefore, there is also a need for a system capable of facilitating the practice of exercises and improving the effectiveness of therapy.

### SUMMARY

The invention thus relates to a system for monitoring at least one uncontrolled behavior, said at least one uncontrolled behavior being preceded by a premonitory urge experienced by a user, said system comprising a sensing device including at least one motion sensor, said motion sensor being configured to deliver a motion signal representative of at least one voluntary action performed by said user in response to said at least one premonitory urge; said system further comprising at least one processor configured to receive said motion signal from said sensing device and to determine, based on said motion signal, said at least one premonitory urge.

In other words, the invention proposes a system dedicated to assisting a user suffering from uncontrolled behaviors in controlling and documenting his premonitory urges and uncontrolled behaviors. The system from the invention may be used to help improving self-control when the user is undergoing acute crisis and/or chronic crisis. The system may also be used as part of a therapy, to practice exposure exercises prescribed by a healthcare professional. To that end, the user utilizes a sensing device that is specially dedicated to the collection of data on the premonitory urges and uncontrolled behaviors.

In that respect, the claimed invention differs from existing solutions, wherein the user is required to collect the data by themself. Indeed, with the system of the invention, the user is only asked to perform a voluntary action that conveys at least one information on the premonitory urge.

The term "voluntary action" is employed as opposed to a reflex or an unvoluntary action that may be part of the uncontrolled behavior. The voluntary action may be a controlled movement such as at least one contraction of at least one muscle of the user (e.g. blinking, clenching one's fist, or moving one's finger in a repetitive manner). The at least one information on the premonitory urge may then be conveyed by varying the duration, the frequency of the force of the voluntary action.

A voluntary action according to the invention is therefore an action that is easy to carry out for the user and that does not require a complex thought process. As a consequence, the user can wholly concentrate on the task of trying to control the premonitory urge. The practice of exposure exercises is therefore greatly facilitated and there is no loss of information as the data are collected and stored by the system and can be directly consulted by the user themself or the healthcare professional.

According to other aspects of the invention, the device comprises one or more of the features described in the following embodiments, taken alone or in any possible combination.

According to one embodiment, said at least one processor is configured to initiate at least one exposure exercise, wherein said exposure exercise comprises exposing said user to at least one stimulus for inducing said at least one uncontrolled behavior, said exposure exercise having a predetermined duration, said motion sensor being configured to deliver said motion signal during said predetermined duration.

According to the invention, the motion signal is representative of the at least one voluntary action performed by the user. Thus, the motion signal may be delivered as many times as the user performs the voluntary action during the predetermined duration. Features of the motion signal such as a number, a duration, a frequency, a pressure may be further analyzed to characterize the premonitory urges.

According to one embodiment, said motion sensor is configured to detect at least one contraction of at least one muscle of said user as the voluntary action.

According to one embodiment, the sensing device is configured to be carried by said user.

According to the present invention, by "carried", it is meant that the user can transport the sensing device on themself as an article of clothing or an accessory. For instance, the sensing device may be in the form of a watch, a bracelet, a pair of glasses, a headset or a bag.

Advantageously, the user can keep the device with themself at all time and perform his exercises whenever he has the opportunity to do so.

According to one embodiment, said sensing device is configured to be hand-held by said user. In that particular embodiment, the sensing device is preferably portable and may be sized so that the user's hand can comfortably wrap around the sensing device.

According to one embodiment, said motion sensor is a pressure sensor configured to detect a pressure exerted by a hand of said user on said sensing device as the voluntary action.

According to one embodiment, said motion sensor is configured to modulate at least one parameter of said motion signal as a function of at least one feature of said at least one premonitory urge, said at least one processor being further configured to determine an evolution of said at least one feature as a function of time based on said motion signal.

In other words, the motion sensor is configured to detect variations of at least one parameter (e.g. duration, force, frequency) of the voluntary action performed by the user. Based on these detected variations, the at least one processor can determine how the premonitory urge affects the user.

According to one embodiment, said at least one feature is at least one of an intensity, a frequency, a force, a pattern and a duration.

According to one embodiment, the at least one processor is configured to instruct said user to perform said at least one voluntary action in response to said at least one premonitory urge.

In some other embodiments, the system may comprise additional features to help the user implement the exposure exercises and keep a steady practice throughout the whole duration of the therapy.

Thus, according to one embodiment, the system may further comprise a sensory feedback device configured to provide to said user an indication of a progression in said predetermined duration of said exposure exercise.

Sensory feedbacks refer to information or signals that the nervous system receives from the sensory organs, such as eyes, ears, skin, muscles, and other sensory receptors. For instance, the sensory feedback device may be a visual feedback, a haptic feedback or a sound.

According to another embodiment, the sensing device may further comprise an actuation member configured to be actuated by said user when said uncontrolled behavior occurs during said predetermined duration of the exposure exercise, the at least one processor being further configured to determine a number of uncontrolled behaviors that occurred during the predetermined duration of the exposure exercise.

In other words, the sensing device may be further configured to count the number of occurrences of an uncontrolled behavior during the exposure exercise. Such information may be cross-referenced with the data collected on the premonitory urge to determine new information such as how the intensity of a premonitory urge influences the occurrence of an uncontrolled behavior, or how the control of a premonitory urge influences in time the occurrence and frequency of an uncontrolled behavior

According to another aspect, the invention relates to a method for monitoring at least one uncontrolled behavior, said uncontrolled behavior being preceded by a premonitory urge experienced by a user, said method comprising:
- delivering a motion signal representative of at least one voluntary action performed by said user in response to said at least one premonitory urge,
- determining, based on said motion signal, said at least one premonitory urge.

According to one embodiment, said method further comprises initiating at least one exposure exercise having a predetermined duration, wherein said exposure exercise comprises exposing said user to at least one stimulus for inducing said at least one uncontrolled behavior and delivering said motion signal during said predetermined duration.

### BRIEF DESCRIPTION OF THE DRAWINGS

**Figure 1** is a schematic representation of the system from the invention according to one embodiment;
**Figure 2** is a schematic representation of a sensing device of the system from Figure 1, according to a first embodiment;
**Figure 3** is a schematic representation of a sensing device of the system from Figure 1, according to a second embodiment; and
**Figure 4** is an exemplary embodiment of a user-interface of the application from the system of Figure 1.

### ILLUSTRATIVE EMBODIMENTS

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

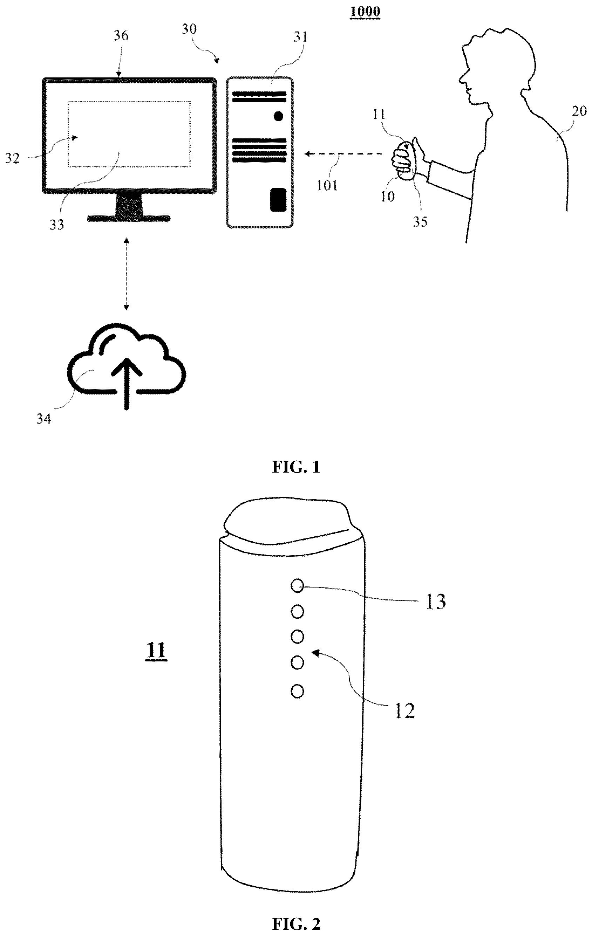

With reference to **Figure 1****,** the present invention relates to a system **1000** for monitoring at least one uncontrolled behavior.

Such system **1000** may be primarily employed by the user **20** for improving his self-control over his premonitory urges and uncontrolled behaviors. The system **1000** may further be implement as part of a therapy, such as a cognitive-behavioral therapy (CBT), to help a user **20** carry on exercises such as exposure exercises wherein the user **20** subjects themself to at least one uncontrolled behavior-provoking situation and tries to control the premonitory urge that precedes the uncontrolled behavior.

According to the invention, uncontrolled behaviors may be compulsive behaviors such as tics, eating disorders, Trichotillomania, Obsessive Compulsive Disorder, Body Dysmorphic Disorder, Tourette Syndrome or addictions including alcoholism, compulsive shopping or gambling, or anxious behavior such as Generalized Anxiety Disorder, Panic Disorder, Post-Traumatic Stress Disorder, phobia including acrophobia, agoraphobia, glossophobia, sociophobia, mysophobia or claustrophobia.

The system **1000** comprises a sensing device **11** configured to work in combination with a dedicated application **32.**

The sensing device **11** is configured to detect at least one voluntary action performed by a user **20** thanks to at least one motion sensor **13.** The motion sensor **13** is further configured to deliver a motion signal **101** characteristic of at least one parameter of the voluntary action during the predetermined duration of the exposure exercise.

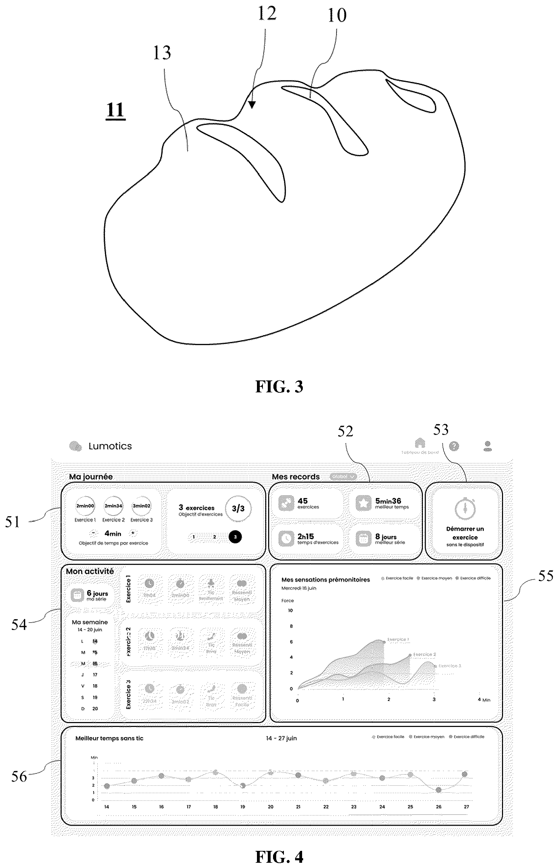

To this end, the sensing device **11** may comprise a body or a plurality of bodies configured to be worn or carried by the user **20** as part of an article of clothing or an accessory. The device **11** is preferable hand-sized to allow the user **20** to exert a comfortable and secured grip on the sensing device **11.** For instance, as illustrated on **Figure 2****,** the sensing device **11** may have an overall tubular shape with a height comprised between 5 cm and 15 cm and a diameter comprised between 3 cm and 7 cm. Alternatively, as illustrated on **Figure 3****,** the sensing device **11** may be potato-shaped with few or no edges to improve ergonomy. The sensing device **11** may be available in different shapes and sizes to best satisfy the user's needs, age or gender.

The sensing device **11** outer shell is preferably made of a material that is resistant to frequent usage, including chocs and sweat and that can be easily cleaned. For instance, the sensing device **11** may be made of plastic, silicone, rubber, stainless steel, aluminum or ceramic.

The sensing device **11** preferably has a weight comprised between 10g and 1kg.

The sensing device **11** may comprise a support zone **12** configured to support the fingers. The support zone **30** may include a plurality of slots **31** which provide a better grip for the user's hand.

The at least one motion sensor **13** may be positioned inside the sensing device **11** or on the outer shell of the sensing device **11.**

The motion sensor **13** may be at least one of an inertial measurement unit (IMU), an accelerometer, a gyroscope, a magnetometer, a tilt sensor, an image sensor such as a camera, a lidar, an infra-red sensor, an ultrasonic sensor, a pressure sensor such as a strain gauge, a piezoelectric sensor, a capacitive sensor or a combination thereof.

In the example of **Figure 2** and **Figure 3****,** the sensing device **11** comprises at least one pressure sensor. The pressure sensors may preferably be positioned in the support zone **12** and configured to detect a pressure exerted by the fingers of the user **20** on said support zone **12.**

The motion sensor **13** may be configured to detect a presence or an absence of the voluntary action. Preferably, the motion sensors **13** are further configured to detect a variation of at least one parameter of the voluntary action performed by the user **20.** To that end the pressure sensor may be a Force Dependent Resistors (FDR), which have the characteristic of varying their outputted value in function of the force or pressure exerted.

In the exemplary case of **Figure 2** and **Figure 3****,** the pressure sensors are preferably configured to detect a variation of a strength of the pressure exerted by the fingers on the sensing device **11.**

The sensing device **11** may further include at least one actuation member **13** such as a switch or a button. Advantageously, the actuation members **13** are positioned so that the user **20** can easily perform both the voluntary action and interact with the actuation member **13.**

One of the actuation members **13** may be configured to switch on and/or switch off the sensing device **11.** Alternatively, the sensing device **11** may automatically switch on when a voluntary action is performed by the user **20.** In the same manner, the sensing device **11** may be configured to switch off when no voluntary action is performed by the user **20** more than a predetermined period comprised between 10 minutes and 1 hour, such as 15 minutes, 30 minutes or 1 hour.

Another actuation members **13** may be actuated to flag the occurrence of an uncontrolled behavior.

Additionally, the sensing device **11** may include at least one sensory feedback device. For instance, the sensing device **11** may include a sensory feedback device configured to convey the state (e.g. on, off, level of battery) of the sensing device **11.** Such sensory feedback device may be a light-emitting device such as a diode, a vibration-emitting device or a sound-emitting device.

The sensing device 11 may also include a sensory feedback device configured to provide an information on the time left to complete the exposure exercise.

Moreover, the sensory feedback device may indicate the time spent without experiencing an uncontrolled behavior while performing the voluntary action.

In the context of the invention, the application **32** typically refers to a software program or a set of programs designed to perform a specific task or set of tasks for end-users. The application **32** may take various forms, and be implemented on dedicated apparatuses or devices to serve specific purposes. For instance, the application **32** may be installed and executed on at least one apparatus **30** such as a personal device of the user **20** (e.g. a computer, a laptop, a smartwatch, a smartphone or a tablet). Alternatively, the application **32** may be installed and executed directly on the sensing device **11.** According to another embodiment, the application **32** may be accessed through web browsers using the apparatus **30** equipped with an internet connectivity or the sensing device **11** equipped with an internet connectivity. In that case, the application **32** is hosted on at least one server **34.** Users **20** may interact with a user-interface **33** of the application **32** through a web browser, and the application **32** processes requests on the at least one server **34.** Advantageously, the application **32** may be hosted on a cloud infrastructure. Alternatively, the application **32** may be a client-server application in which the processing responsibilities are distributed between the apparatus **30** or sensing device **11** on which the application **32** is installed and the server **34** (e.g. a remote computer or cloud server).

The application **32** may be executed by a single dedicated processor, a single shared processor, or a plurality of individual processors, some of which may be shared. For instance, the application **32** may be entirely executed by at least one processor **35** included inside the sensing device **11** or at least one processor **31** included inside the apparatus **30.** Processing may also be shared between the at least one processor **35** included in the sensing device **11** and the at least one processor **31** included inside the apparatus **30.**

Alternatively, the application **32** may be at least partially executed on at least one remote processor included on the at least one server **34.**

To be able to run the application **32,** the sensing device **11** and/or the apparatus **30** may include a memory and input/output interfaces. The apparatus **30** and/or the sensing device **11** may comprise at least part of the following elements, connected to each other by a bus of addresses and data that also transports a clock signal:
- a microprocessor (or CPU);
- a graphics card comprising several Graphical Processing Units (or GPUs) and a Graphical Random Access Memory (GRAM); the GPUs are quite suited to image processing, due to their highly parallel structure;
- a non-volatile memory of ROM type;
- a RAM;
- one or several I/O (Input/Output) devices such as for example a keyboard, a mouse, a trackball, a webcam; other modes for introduction of commands such as for example vocal recognition are also possible;
- a power source; and
- a radiofrequency unit.

According to a variant, the power supply may be external to the apparatus **30** and/or the sensing device **11.**

The apparatus **30** and/or the sensing device **11** may also comprise a display device **36** directly connected to the graphics card to display synthesized images calculated and composed in the graphics card. According to a variant, the display device is external and is connected to the apparatus **30** and/or the sensing device **11** by a cable or wirelessly for transmitting the display signals. The apparatus **30** and/or the sensing device **11,** for example through the graphics card, comprises an interface for transmission or connection adapted to transmit a display signal to an external display means such as for example an LCD or plasma screen or a video-projector. In this respect, the RF unit can be used for wireless transmissions.

The apparatus **30** and/or the sensing device **11** may also comprise at least one memory. It is noted that the word "memory" used in the description of memories can designate in each of the memories mentioned, a memory zone of low capacity (some binary data) as well as a memory zone of large capacity (enabling a whole program to be stored or all or part of the data representative of data calculated or to be displayed). Also, the memories represented for the RAM and the GRAM can be arranged and constituted in any manner, and each of them does not necessarily correspond to adjacent memory locations and can be distributed otherwise (which covers notably the situation in which one register includes several smaller registers).

When switched-on, the microprocessor loads and executes the instructions of the program contained in the RAM.

As will be understood by a skilled person, the presence of the graphics card is not mandatory, and can be replaced with entire CPU processing and/or simpler visualization implementations.

The application **32** may be configured to initiate a pairing with the sensing device **11.** To that end, the application **32** may search for available devices, or the sensing device **11** may make itself visible to nearby applications or devices. This may involve Bluetooth, Wi-Fi, NFC (Near Field Communication), or other wireless communication methods.

The application 32 may also include a feature for launching 53 a exposure exercise. To that end, the application 32 and/or the sensing device 11 may comprise a "Start," "Begin," or similar button to initiate the exposure exercise.

The application **32** may include several additional features illustrated on **Figure 4****.**

For instance, the application **32** may comprise a personalization phase wherein the user **20** can specify generic information such as a name, gender, age, or type of uncontrolled behavior.

The application may also include a scheduling feature **51** for scheduling the exposure exercises. The user **20** may schedule a type of exposure exercise, a level of difficulty, a number of exposure exercise, a duration of the exposure exercise, a location of the exposure exercise and a frequency of said exposure exercises (e.g. time of the day, daily occurrence, weekly occurrence).

The application **32** may include an alarm feature to remind the user **20** to perform his exposure exercise at the scheduled times.

Additionally, the application **32** may include at least one feature allowing to follow-up an evolution in the course of the therapy.

For instance, the application **32** may include a summary of an activity of the user **20,** including a visualization of data on each exposure exercise performed. Such data may include the time and day at which the exposure exercise was launched, the actual duration of the exposure exercise, the number and type of uncontrolled behavior that occurred. Such data may be compared to what was initially scheduled.

The application **32** may also comprise a comparative graphic representation **55** of an evolution of an intensity of the premonitory urge as a function of time for each exposure exercise performed by the user **20.**

The application **32** may also comprise a graphic representation **56** of the evolution of a duration of a premonitory urge with no uncontrolled behavior occurrence. This feature allows the user **20** to visualize the habituation process.

The application **32** may also propose challenges to the user **20** to motivate him in performing the exposure exercises. The application **32** may therefore include a record-tracking feature **52** to highlight at least one of the number of exposure exercises performed, the total timed spent in doing the exposure exercises, the total number of consecutive days where the user **20** did perform his exposure exercises.

Further to the realization of an exposure exercise, the user **20** may be asked to complete a questionary. For instance, the user **20** may be asked to grade the difficulty in realizing the exposure exercise and/or to indicate if an uncontrolled behavior occurred during the exposure exercise and the number of uncontrolled behaviors that occurred during the exposure exercise. The data obtained from the questionary may be compared with the data collected by the sensing device **11.**

The application **32** may also propose information and recommendations to better understand the symptoms of the disease associated with the uncontrolled behavior, the purpose of therapy and how to explain the disease to other people.

Optionally, the application **32** may propose at least one initiation tutorial to help the user **20** famifiarize themself with the different features of the application **32.**

The different features proposed by the application may be gathered on a dashboard for the user **20** to have immediate access to above-mentioned features.

In use, when the user **20** wishes to perform an exercise, he takes the sensing **11** device in his hand and performs the exercise by squeezing the device more or less tightly, depending on the intensity of his premonitory urge.

Afterward, the user **20** can track his progress on the application **32** thanks to the data collected by the sensing device **11** and details that may be additionally provided by the user **20** themself through questionaries.

Advantageously, the healthcare professional may also have access to the user's progress and adapt the therapy by interpretating the data collected by the sensing device **11.**

## Claims

1. A system (1000) for monitoring at least one uncontrolled behavior, said at least one uncontrolled behavior being preceded by at least one premonitory urge experienced by a user (20), said system (1000) comprising a sensing device (11) including at least one motion sensor (13), said motion sensor (13) being configured to deliver a motion signal (101) representative of at least one voluntary action performed by said user (20) in response to said at least one premonitory urge; said system (1000) further comprising at least one processor (30) configured to receive said motion signal (101) from said sensing device (11) and to determine, based on said motion signal (101), said at least one premonitory urge.

2. The system according to claim **1,** wherein said at least one processor (30) is configured to initiate at least one exposure exercise, wherein said exposure exercise comprises exposing said user (20) to at least one stimulus for inducing said at least one uncontrolled behavior, said exposure exercise having a predetermined duration, said motion sensor (13) being configured to deliver said motion signal (101) during said predetermined duration.

3. The system according to claim **1** or **2,** wherein said sensing device (11) is configured to be carried by said user (20).

4. The system according to any of claims **1** to **3,** wherein said sensing device (11) is configured to be hand-held by said user (20).

5. The system according to any of claim **1** to **4,** wherein said motion sensor (11) is configured to detect at least one contraction of at least one muscle of said user (20) as the voluntary action.

6. The system according to any of claim **1** to **5,** wherein said motion sensor (11) is a pressure sensor configured to detect a pressure exerted by a hand of said user (20) on said sensing device (11) as the voluntary action.

7. The system according to any of claims **1** to **6,** wherein said motion sensor (11) is configured to modulate at least one parameter of said motion signal (101) as a function of at least one feature of said at least one premonitory urge, said at least one processor (30) being further configured to determine an evolution of said at least one feature as a function of time based on said motion signal (101).

8. The system according to claim **7,** wherein said at least one feature is at least one of an intensity, a frequency, a force, a pattern and a duration.

9. The system according to any of claims **1** to **8,** wherein the at least one processor (30) is configured to instruct said user (20) to perform said at least one voluntary action in response to said at least one premonitory urge.

10. The system according to any of claims **1** to **9,** wherein the system (1000) further comprises a sensory feedback device configured to provide to said user (20) an indication of a progression in said predetermined duration of said exposure exercise.

11. The system according to any of claims **1** to **10,** wherein said sensing device (11) further comprises an actuation member (13) configured to be actuated by said user (20) when said uncontrolled behavior occurs during said predetermined duration of the exposure exercise, the at least one processor (30) being further configured to determine a number of uncontrolled behaviors that occurred during the predetermined duration of the exposure exercise.

12. A method for monitoring at least one uncontrolled behavior, said uncontrolled behavior being preceded by a premonitory urge experienced by a user (20), said method comprising:
- delivering a motion signal (101) representative of at least one voluntary action performed by said user (20) in response to said at least one premonitory urge,
- determining, based on said motion signal (101), said at least one premonitory urge.

13. The method from claim **12,** wherein said method further comprises initiating at least one exposure exercise having a predetermined duration, wherein said exposure exercise comprises exposing said user (20) to at least one stimulus for inducing said at least one uncontrolled behavior and delivering said motion signal (101) during said predetermined duration.
